# EUROPEAN PATENT APPLICATION

(11) **EP 4 650 344 A1**
(43) Date of publication of application: **19.11.2025**
(21) Application number: 24753608.9
(22) Date of filing: 06.02.2024
(51) Int. Cl.: C07C 319/28, C07C 323/58, C12P 13/12

(54) **METHOD FOR PREPARING L-CYSTEINE HYDROCHLORIDE HYDRATE CRYSTALS OF WHICH PARTICLE SIZE IS CONTROLLED**

(30) Priority: 07.02.2023 KR 20230016329
(71) Applicant: CJ CheilJedang Corporation, Seoul 04560 (KR)
(72) Inventor: KIM, Jungwon, Seoul 04560 (KR); JUNG, Jun Young, Seoul 04560 (KR); LEE, In Sung, Seoul 04560 (KR); PARK, Shin-ae, Seoul 04560 (KR); HONG, Je-won, Seoul 04560 (KR)
(74) Representative: Meissner Bolte Partnerschaft mbB
(86) International application number: PCT/KR2024/001741
(87) International publication number: WO 2024/167283

(57) **Abstract**

The present disclosure relates to a method for producing L-cysteine hydrochloride hydrate crystals having a controlled particle size, and L-cysteine hydrochloride hydrate crystals having a controlled particle size produced by the production method.

## Description

### [Technical Field]

The present disclosure relates to a method for producing L-cysteine hydrochloride hydrate crystals having a controlled particle size.

### [Background Art]

L-cysteine is an amino acid that plays an important role in sulfur metabolism in all living organisms. It is used in the biosynthesis of proteins such as hair keratin, glutathione, biotin, methionine, and other sulfur-containing metabolites, and also as a precursor of coenzyme A. Additionally, L-cysteine and its derivatives can be industrially used in various fields, including the pharmaceutical industry for the treatment of bronchial diseases, *etc.,* the cosmetic industry for hair shampoos, permanent compositions, *etc.,* and the food industry for antioxidants and flavor enhancers, *etc.*

L-cysteine is generally produced by decomposing animal-derived L-cysteine, which uses duck feathers or human hair as a source material, or fermentation-derived L-cysteine, which uses a microbial metabolism liquid as a source material, into L-cysteine by an electrochemical reduction reaction. Recently, methods for producing L-cysteine using microorganisms, including a process for producing natural L-cysteine by fermentation using a strain having a modified O-acetyl transferase in a medium containing sulfide (US8802399B, US6946268B), and a process for producing natural L-cysteine by mixing O-phosphohomoserine produced by a microbial culture method with sulfide and inducing an enzyme catalytic reaction using O-phosphoserine sulfhydrylase (WO2013/089478, WO2012/053794), *etc.* are also actively carried out.

In the food industry, the fine particle size of L-cysteine hydrochloride is considered important to facilitate mixing with other raw materials. However, in conventional technologies, L-cysteine hydrochloride has been produced with a wide particle size range in which the ratio of fine particles of 40 mesh or less is less than 20%. Thus, since the particle ratio was low and the sizes of the obtained L-cysteine hydrochloride hydrates were different from each other, the introduction of an additional sieving process was required to obtain L-cysteine hydrochloride hydrates of a desired particle size. Additionally, the sieving process significantly increased manpower, initial investment cost, and energy consumption cost due to the low particle ratio of the produced particles, which was a problem. In some conventional processes, the particle size of L-cysteine hydrochloride hydrates was controlled by adjusting the stirring speed during the production process, but there was a limitation that the effect of controlling the particle size was not significant.

Accordingly, since L-cysteine hydrochloride hydrate crystals are applied to various fields, including the pharmaceutical industry, food industry, and cosmetics industry, as described above, there is a need to adjust the particle size specifications of L-cysteine hydrochloride hydrate crystals for uniform mixing of raw materials and the efficacy of active ingredients during the production of application products, and in particular, the development of a method for producing micronized L-cysteine hydrochloride hydrate with a high particle size ratio of 40 mesh or less is one of the technical challenges to be solved in the art.

### [Disclosure]

### [Technical Problem]

It is one object of the present disclosure to provide a method for producing L-cysteine hydrochloride hydrate crystals having a controlled particle size, including:
(a) an initial hydrochloric acid addition step for adjusting the initial molar ratio of hydrochloric acid to L-cysteine (HCI/L-cysteine) by adding hydrochloric acid to a solution containing L-cysteine;
(b) a concentration step for concentrating the solution to which the hydrochloric acid has been added to obtain a concentrate;
(c) an additional hydrochloric acid addition step for adjusting the final molar ratio of hydrochloric acid to L-cysteine (HCI/L-cysteine) by additionally adding hydrochloric acid to the concentrate; and
(d) a recovering step for recovering L-cysteine hydrochloride hydrate crystals from the concentrate to which hydrochloric acid has been additionally added.

It is another object of the present disclosure to provide L-cysteine hydrochloride hydrate crystals produced by the production method above.

### [Technical Solution]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Additionally, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a method for producing L-cysteine hydrochloride hydrate crystals having a controlled particle size, including:
(a) an initial hydrochloric acid addition step for adjusting the initial molar ratio of hydrochloric acid to L-cysteine (HCI/L-cysteine) by adding hydrochloric acid to a solution containing L-cysteine;
(b) a concentration step for concentrating the solution to which the hydrochloric acid has been added to obtain a concentrate;
(c) an additional hydrochloric acid addition step for adjusting the final molar ratio of hydrochloric acid to L-cysteine (HCI/L-cysteine) by additionally adding hydrochloric acid to the concentrate; and
(d) a recovery step for recovering L-cysteine hydrochloride hydrate crystals from the concentrate to which the hydrochloric acid has been additionally added

In one embodiment of the present disclosure, the method may further include a cooling step for cooling the concentrate between the (b) concentration step and the (c) additional hydrochloric acid addition step, concurrently with the (c) additional hydrochloric acid addition step, or after the (c) additional hydrochloric acid addition step.

First, as used herein, "L-cysteine", which is a type of alpha amino acid and is an L-amino acid with the chemical formula of HO₂CCH(NH₂)CH₂SH, is the only sulfur-containing amino acid having a thiol group (R-SH) among L-amino acids. L-Cysteine may be obtained by chemical synthesis, or biological synthesis through microbial fermentation, *etc.,* but is not limited thereto. Specifically, in the present disclosure, L-cysteine may be L-cysteine biologically produced through microbial fermentation, or may be natural L-cysteine obtained by inducing an enzyme catalytic reaction of O-phosphohomoserine, which is a precursor prepared through microbial fermentation, with a sulfide in the presence of phosphoserine sulfhydrylase, but is not limited thereto.

As used herein, the term "L-cysteine monohydrochloride" refers to a colorless or white crystal with a unique odor and taste with the chemical formula C₃H₇NO₂SHCl.H₂O, and is a stable form compared to the relatively unstable L-cysteine.

As used herein, the term "fermentation broth" refers to a culture medium obtained by culturing L-cysteine-producing microorganisms, a culture containing the microorganisms cultured together with the culture medium, or an enzyme conversion solution containing a precursor capable of producing L-cysteine and an enzyme. Specifically, the fermentation broth containing L-cysteine may be a culture medium or an enzyme conversion solution containing natural L-cysteine. More specifically, it may be an L-cysteine culture or culture broth biologically prepared by fermenting microorganisms having an L-cysteine-producing ability, or may be a natural L-cysteine enzyme conversion solution obtained by inducing an enzyme catalytic reaction of O-phosphohomoserine, which is a precursor prepared through microbial fermentation, with a sulfide in the presence of phosphoserine sulfhydrylase, but is not limited thereto.

In the present disclosure, in the method for producing L-cysteine hydrochloride hydrate crystals, it was found that adjusting the molar ratio of hydrochloric acid to L-cysteine twice, before and after concentration, and adjusting the initial molar ratio and the final molar ratio of hydrochloric acid to L-cysteine to a specific range are entirely characteristic factors in controlling the particle size of the finally obtained L-cysteine hydrochloride hydrate crystals.

Therefore, there is a technical feature confirming that the present disclosure provides L-cysteine hydrochloride hydrate crystals, in which the particle size is controlled such that the ratio of fine crystals is significantly increased, *i.e.,* the ratio of fine particles of 40 mesh or less, especially the ratio of particles having a uniform particle size of 40 to 80 mesh, is significantly increased, by adjusting the molar ratio of hydrochloric acid to L-cysteine twice for the initial molar ratio (HCI/L-cysteine) and the final molar ratio (HCl/L-cysteine).

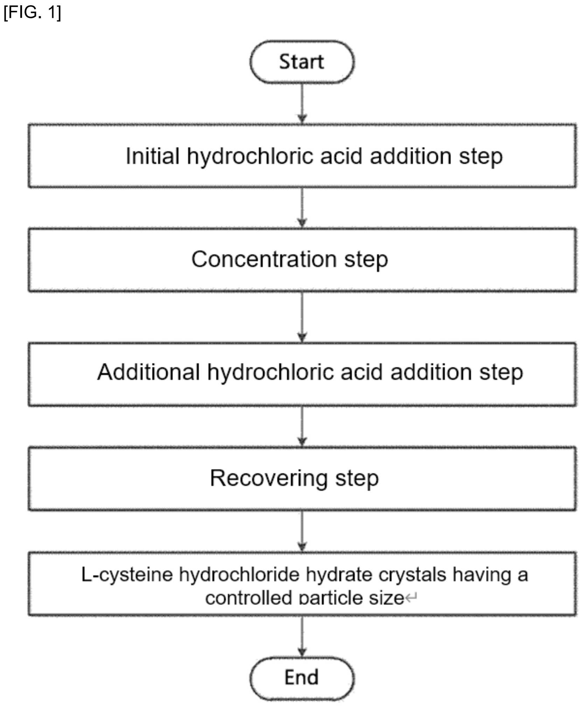

FIG. 1 shows a flowchart showing the production method according to one embodiment of the present disclosure.

Hereinafter, the production method of L-cysteine hydrochloride hydrate crystals having a controlled particle size according to the present disclosure will be described in detail according to each step.

The (a) initial hydrochloric acid addition step refers to a step of adjusting the initial molar ratio of hydrochloric acid to L-cysteine (HCI/L-cysteine) by adding hydrochloric acid to a solution containing L-cysteine.

In one embodiment of the present application, the solution containing L-cysteine may be a fermentation broth containing L-cysteine, and in particular, the method for producing L-cysteine hydrochloride hydrate crystals having a controlled particle size according to the present disclosure may further include a purification step of obtaining a separated solution after injecting the fermentation broth containing L-cysteine into a chromatography device before the (a) initial hydrochloric acid addition step. Specifically, the chromatography device may be continuous chromatography, but is not limited thereto.

As used herein, the term "mole fraction" means the ratio of the number of gram molecules of the constituting component to the number of gram molecules of the whole mixture and is also called the mole proportion or the molar proportion. In one example, in the present disclosure, the "molar ratio" of hydrochloric acid to L-cysteine represents the number of moles of HCl/the number of moles of L-cysteine. In the present disclosure, it has been found that the particle size of L-cysteine hydrochloride hydrate crystals, which can be obtained by concentrating the solution, can be adjusted by adjusting the molar ratio of hydrochloric acid to L-cysteine, which is a technical feature.

Specifically, the (a) initial hydrochloric acid addition step may be a step of adding hydrochloric acid to a solution containing L-cysteine such that the initial molar ratio of hydrochloric acid to L-cysteine (HCI/L-cysteine) is 0.5 or more and less than 1.1, and more specifically, it may be a step of adding hydrochloric acid such that the initial molar ratio (HCI/L-cysteine) is 0.6 or more and 1.0 or less, 0.6 or more and 0.9 or less, 0.7 or more and 1.0 or less, 0.75 or more and 0.95 or less, 0.8 or more and 0.95 or less, 0.85 or more and less than 1.0, or 0.8 or more and less than 1.0. When the initial molar ratio (HCI/L-cysteine) is adjusted within the above range, the ratio of particles having a particle size of 40 to 80 mesh in the finally obtained L-cysteine hydrochloride hydrates increases, and thus, L-cysteine hydrochloride hydrate crystals having a controlled particle size can be easily obtained. Even when the final molar ratio (HCI/L-cysteine) is adjusted to the same value, if the initial molar ratio is too low compared to the above range, the crystal yield in the finally produced L-cysteine hydrochloride hydrates may be significantly low, and if the initial molar ratio is too high, the ratio of particles having a particle size of 40 to 80 mesh may be significantly low.

The (b) concentration step is a step for obtaining a concentrate by concentrating a solution to which hydrochloric acid has been added, and is a step for the crystallization of L-cysteine hydrochloride hydrate.

In step (b), the separated solution to which hydrochloric acid has been added can be concentrated to obtain a concentrate. The concentration can be performed in a conventional concentrator (*e.g.,* a forced circulation concentrator, a thin film concentrator, or a rotary concentrator, etc.), which may be selected by those skilled in the art. The concentration of L-cysteine in the concentrate in step (b) may range from 100 to 900 g/L, specifically 300 to less than 800 g/L, 400 to less than 800 g/L, 400 to less than 700 g/L, and more specifically 500 to less than 700 g/L.

If the concentration of L-cysteine in the concentrate is low, crystallization does not occur during the concentration process due to a lack of supersaturation required for nucleation and crystal growth, and crystallization may be achieved during cooling, or may be carried out through an additional process. However, the recovery rate may be low, or the crystallization time may be prolonged. If the concentration of L-cysteine in the concentrate is 300 g/L or more, crystal nuclei of L-cysteine hydrochloride hydrates may form during the concentration process. Additionally, when the L-cysteine hydrochloride hydrate slurry concentrated to 300 g/L or more is cooled, the recovery rate of the crystallization process for the L-cysteine hydrochloride hydrates may increase. When the concentration of the L-cysteine hydrochloride hydrates of the concentrate is 900 g/L, solidification may occur due to the formation of a large amount of crystal particles, and accordingly, stirring of the crystal slurry and separation of the crystals may not be possible. Therefore, the step of concentrating the separated solution to which hydrochloric acid has been added may be a step of concentrating the solution such that the concentration of L-cysteine is 300 g/L to less than 800 g/L, specifically 400 to less than 800 g/L, more specifically 400 to less than 700 g/L, and even more specifically 500 to 700 g/L.

The crystallization of L-cysteine hydrochloride hydrates may occur during the concentration according to the step (b). As used herein, the term "crystallization" refers to a phenomenon by which a liquid or a solid in an amorphous state forms a crystal, and it is accompanied by two phenomena called nucleation and crystal growth.

The (c) additional hydrochloric acid addition step refers to a step of adjusting the final molar ratio of hydrochloric acid to L-cysteine (HCI/L-cysteine) by additionally adding hydrochloric acid to the concentrate.

Specifically, the (c) additional hydrochloric acid addition step may be a step of adding hydrochloric acid such that the final molar ratio (HCI/L-cysteine) of hydrochloric acid to L-cysteine in the solution containing L-cysteine is 0.9 or more and less than 1.5, but additional hydrochloric acid is added in the (c) additional hydrochloric acid addition step, the final molar ratio (HCI/L-cysteine) is a value exceeding the initial molar ratio (HCI/L-cysteine) adjusted in step (a). More specifically, hydrochloric acid may be added such that the final molar ratio (HCI/L-cysteine) is 1.0 or more and less than 1.5, more specifically 1.0 or more and 1.1 or less, 1.05 or more and less than 1.2, 1.0 or more and 1.3 or less, or 1.1 or more and less than 1.2. In one embodiment of the present disclosure, the ratio of particles having a particle size of 40 to 80 mesh was increased in the L-cysteine hydrochloride hydrates finally obtained in the range of a molar ratio of 1.0 or more and less than 1.5, thus confirming that L-cysteine hydrochloride hydrate crystals having a controlled particle size could be easily obtained. Additionally, if the final molar ratio was below the range, the recovery rate of the crystallization process decreased or free form crystals were formed, which were problems, whereas if the final molar ratio exceeded the above range, the ratio of large particles having a particle size of 40 mesh or less increased rapidly. That is, when the molar ratio is adjusted by additionally adding hydrochloric acid once the concentration step is completed after the formation of free-form crystals, the recovery rate of L-cysteine hydrochloride crystals can be increased.

In one embodiment of the present disclosure, a cooling step of cooling the concentrated liquid to which hydrochloric acid has been added may be further included selectively before, after, or concurrently with the (c) additional hydrochloric acid addition step. The concentrate may form and/or grow crystal nuclei through cooling and/or aging prior to recovery by the cooling step. Further, even when the L-cysteine hydrochloride hydrate crystals are not precipitated from the concentrate, crystals may be formed during cooling or aging of the concentrate.

The cooling step may specifically refer to cooling to a temperature of -10 to 55°C over a period of 2 hours to 6 hours, specifically cooling to a temperature of 0 to 45°C over a period of 2 hours to 6 hours, more specifically cooling to a temperature of 0 to 30°C, and even more specifically cooling to a temperature of 0 to 15°C over a period of 2 hours to 6 hours. The aging step may refer to allowing to stand without changing the temperature. In the present disclosure, the cooling step may refer to constantly maintaining the cooled temperature, or it may refer to constantly maintaining the temperature of the concentrate even when it is not cooled.

The (d) recovering step may be a step for recovering L-cysteine hydrochloride hydrate crystals from the concentrate to which the hydrochloric acid has been additionally added. Specifically, the recovery step may be a step for recovering L-cysteine hydrochloride hydrate crystals from the slurry by subjecting the concentrate, to which the hydrochloric acid has been additionally added, to a solid-liquid separation. The solid-liquid separation may be carried out using a solid-liquid separator such as a reduced-pressure membrane filtration apparatus, a pressure membrane filtration apparatus, a centrifugal separation apparatus, *etc.,* but is not limited thereto. In one embodiment of the present disclosure, a step of additionally washing or drying the slurry and/or precipitated L-cysteine crystals may be further included additionally after the (d) step.

In the present disclosure, the filtrate obtained by recovering the crystals in the (d) step is a mother liquid having residual L-cysteine, and may be recycled by wholly or partially adding the solution, the solution to which hydrochloric acid has been added, and the concentrate in the (a), (b), and (c) steps, respectively, in order to enhance the recovery rate during the final purification of L-cysteine, but is not limited thereto.

The L-cysteine hydrochloride hydrate prepared according to the method for producing L-cysteine hydrochloride hydrate crystals having a controlled particle size of the present disclosure may be a monohydrate.

Specifically, the L-cysteine hydrochloride hydrate crystals having a controlled particle size prepared according to the production method of the present disclosure may have a ratio of micronized particles having a particle size of 40 mesh or less of 50% or more and less than 90%, more specifically 55% or more and less than 90%, 57% or more and less than 90%, 60% or more and less than 90%, 62% or more and less than 90%, 65% or more and less than 90%, or 68% or more and less than 80%.

Further, specifically, the L-cysteine hydrochloride hydrate crystals having a controlled particle size prepared according to the production method of the present disclosure may have an increased ratio of particles having a particle size of 40 to 80 mesh. More specifically, the L-cysteine hydrochloride hydrate crystals having a controlled particle size prepared according to the production method of the present disclosure may have a ratio of particles having a particle size of 40 to 80 mesh of 50% or more and less than 90%, more specifically 52% or more and less than 90%, 55% or more and less than 90%, 60% or more and less than 90%, 62% or more and less than 90%, or 65% or more and less than 80%. The particle size of 40 to 80 mesh is a size of a small fine particle but is not a size of a micro fine particle that reduces the efficiency of production process. Thus, it is a preferred range of particle size of L-cysteine hydrochloride crystals for production of products in the fields of cosmetics, foods and pharmaceuticals, because within the range, L-cysteine hydrochloride crystals can be easily mixed with other raw materials and can be easily transported during the production process without causing problems of process efficiency due to the generation of dust particles. The L -cysteine hydrochloride hydrate crystals prepared according to the production method of the present disclosure can be applied in various industrial fields because the ratio of particles having a particle size of 40 to 80 mesh was significantly increased by dual adjustment of molar ratios.

As used herein, the mesh is one of the units representing the size of the openings of sieve or powder particle, and the mesh means the number of mesh openings contained in a square with one side of 1 inch (25.4 mm) according to the Tyler Standard Sieve. In the present disclosure, particles having a particle size equal to or smaller than a specific mesh are interpreted to include particles having the same particle size as the specific mesh and particles having a particle size smaller than the specific mesh, and particles having a particle size equal to or greater than a specific mesh are interpreted to include particles having the same particle size as the specific mesh and particles having a particle size larger than the specific mesh.

Accordingly, the L-cysteine hydrochloride hydrate crystals having a controlled particle size prepared according to the production method of the present disclosure may have a particle size of 50% or more of the total number of particles in the range of 0.180 mm to 0.425 mm, and more specifically a particle size of 52% or more and less than 90% of the total number of particles, 55% or more and less than 90% of the total number of particles, 60% or more and less than 90% of the total number of particles, 62% or more and less than 90% of the total number of particles, or 65% or more and less than 80% of the total number of particles may be in the range of 0.180 mm to 0.425 mm.

Another aspect of the present disclosure provides L-cysteine hydrochloride hydrate crystals having a controlled particle size, which are prepared according to the method for producing L-cysteine hydrochloride hydrate crystals having a controlled particle size as described above.

In particular, the L-cysteine hydrochloride crystals are characterized by an increased ratio of particles having a particle size of 40 to 80 mesh compared to L-cysteine hydrochloride hydrate crystals produced by a conventional production method. Specifically, the L-cysteine hydrochloride crystals may have a ratio of particles having a particle size of 40 to 80 mesh increased by 5 to 50% compared to L-cysteine hydrochloride hydrate crystals produced by a conventional manufacturing method.

The characteristics and composition of the L-cysteine hydrochloride hydrate crystals and the method for producing the same are as described above.

### [Advantageous Effects]

The method for producing L-cysteine hydrochloride hydrate crystals having a controlled particle size of the present disclosure can provide L-cysteine hydrochloride hydrate crystals having a controlled particle size through dual adjustment of the hydrochloric acid/cysteine molar ratio. In particular, since the ratio of micronized crystal particles having a particle size of 40 to 80 mesh is increased, manpower, initial investment cost, and energy consumption cost for the sieving process can be significantly reduced, and the uniformity and efficacy of effective ingredients in products applied in various fields such as cosmetics, foods, and pharmaceuticals can be improved.

### [Brief Description of Drawing]

FIG. 1 is a diagram showing the progress of the method for producing L-cysteine hydrochloride hydrate crystals having a controlled particle size according to one embodiment of the present disclosure.

### [Mode for Carrying Out the Invention]

Hereinafter, the present disclosure will be described in detail by way of Examples. However, these Examples are merely preferred Examples given for illustrative purposes, and thus, the scope of the invention is not intended to be limited to or by these Examples.

### Example 1: Confirmation of Crystal Form by Molar ratio of Hydrochloric Acid/Cysteine

In order to confirm the crystal form of L-cysteine, the crystal form of L-cysteine obtained by concentrating and cooling the solution according to the hydrochloric acid/cysteine molar ratio was confirmed. Hydrochloric acid was added to a solution containing L-cysteine at hydrochloric acid/cysteine molar ratios of 0.5, 0.8, 0.85, 0.9, and 1.0, and the pH of the solution after the addition was between 0.4 and 2.0. When the molar ratio of hydrochloric acid/cysteine was less than 0.9, the crystals formed during the concentration or cooling step after concentration were in the L-cysteine free form, and when the molar ratio of hydrochloric acid/cysteine exceeded 0.9, L-cysteine hydrochloride hydrate crystals were formed.

### Example 2: Confirmation of Particle Size of L-Cysteine Hydrochloride Hydrate Crystals by Molar ratio of Hydrochloric Acid/Cysteine

First, L-cysteine hydrochloride hydrate was produced by the following method to confirm the particle size of L-cysteine hydrochloride hydrate crystals by molar ratio of hydrochloric acid/cysteine.

### (1) Concentration

The concentration was performed under a vacuum of 110 mmHg and an external temperature of 70°C until the concentration of L-cysteine reached 600 g/L. When the molar ratio of hydrochloric acid/cysteine was less than 0.9, L-cysteine free form crystals were precipitated during the concentration, and the temperature of the L-cysteine concentrate immediately after the concentration or crystal slurry was 55 to 60°C.

### (2) Cooling and Recovery of Crystals

The thus-obtained L-cysteine concentrate or crystal slurry was cooled to 15°C at a constant cooling rate for 8 hours in a jacketed tank with stirring. Thereafter, L-cysteine crystals were separated into solid and liquid from the L-cysteine crystal slurry using a basket separator at a rotation speed of 3,000 rpm for 15 minutes. After separation, drying was performed using an oven dryer at 40°C for 2 hours or more to reduce the residual moisture to 0.5% or less, thereby finally producing L-cysteine hydrochloride hydrate crystals. The particle size distribution is shown in Table 1.

**[Table 1]**

| Molar ratio of HCI/ L-cysteine | Particle size of L-cysteine hydrochloride hydrate products (mesh) | | |
|---|---|---|---|
| Eq. | -40 | 40 - 80 | 80 - |
| 0.5 | Formation of L-cysteine Free Form | | |
| 0.8 | Formation of L-cysteine Free Form | | |
| 0.9 | L-cysteine recovery rate decreased | | |
| 1.0 | 36.3 | 63.7 | 0.0 |
| 1.1 | 48.0 | 51.9 | 0.1 |
| 1.2 | 65.4 | 33.7 | 0.9 |
| 1.5 | 82.5 | 17.5 | 0.0 |

As shown in Table 1 above, when L-cysteine hydrochloride hydrate crystals were produced by reaching the final molar ratio only through the initial adjustment, L-cysteine free form crystals were produced. These are a type of crystal formed solely by cysteine when the molar ratio of hydrochloric acid/cysteine was less than 0.9. Additionally, when the molar ratio of hydrochloric acid/cysteine was 0.9 or less, the L-cysteine recovery rate decreased excessively to 20% or less. The L-cysteine recovery rate could only be stably maintained at 45% or more when the final molar ratio of hydrochloric acid/cysteine was 1.0 or higher. Meanwhile, when the molar ratio of hydrochloric acid/cysteine reached 1.2 or higher, the proportion of crystals exceeding 40 mesh increased to 65% or more.

### Example 3: Changes in Crystal Particle Size According to Initial Hydrochloric Acid/Cysteine Molar ratio by Dual Adjustments of Molar ratio

In Example 2, L-cysteine hydrochloride hydrates were produced by additionally adding hydrochloric acid during the concentration step or the cooling step after concentration, under conditions where L-cysteine free form crystals were formed due to an insufficient initial molar ratio of hydrochloric acid/cysteine.

### (1) Concentration

The concentration was performed under a vacuum of 110 mmHg and an external temperature of 70°C until the concentration of L-cysteine reached 600 g/L. When the molar ratio of hydrochloric acid/cysteine was less than 0.9, L-cysteine free form crystals were precipitated during concentration, and the temperature of the L-cysteine concentrate immediately after concentration or crystal slurry was 55 to 60°C.

### (2) Additional Hydrochloric Acid Addition

Hydrochloric acid was added to the L-cysteine concentrate or crystal slurry solution so that the final molar ratio of hydrochloric acid/cysteine became 1.0.

### (3) Cooling and Recovery of Crystals

The thus-obtained hydrochloric acid mixture was cooled to 15°C at a constant cooling rate for 8 hours in a jacketed tank with stirring. Thereafter, L-cysteine crystals were separated into solid and liquid phases from the L-cysteine crystal slurry using a basket separator at a rotation speed of 3,000 rpm for 15 minutes. After separation, drying was performed using an oven dryer at 40°C for 2 hours or more to reduce the residual moisture to 0.5% or less, thereby finally producing L-cysteine hydrochloride hydrate crystals. Regardless of the hydrochloric acid/cysteine molar ratio, the purity of the L-cysteine hydrochloride monohydrate crystals was 99.0% or higher, and the particle size distribution is shown in Table 2 below.

**[Table 2]**

| Initial hydrochloric acid/cysteine molar ratio | Formation of crystal nuclei | Addition al hydroch loric acid | Final hydrochloric acid/cysteine molar ratio | Product mesh | | |
|---|---|---|---|---|---|---|
| Eq. | | Eq. | Eq. | -40 | 40 - 80 | 80 - |
| 0.5 | During concentrati on | 0.5 | 1.0 | 46.2 | 50.8 | 3.0 |
| 0.85 | During concentrati on | 0.15 | 1.0 | 29.3 | 66.7 | 4.0 |
| 1.0 | During cooling | 0.2 | 1.2 | 58.5 | 40.9 | 0.6 |

In all trials, the particle size of 40 to 80 mesh in L-cysteine hydrochloride monohydrate crystals exceeded 50%. It was confirmed that using the method described in the present disclosure allows for obtaining very high-purity L-cysteine hydrochloride monohydrate fine crystals. In particular, it was confirmed that when the initial hydrochloric acid/cysteine molar ratio was adjusted to 0.85, the ratio of the particle size of 40 to 80 mesh in the products significantly increased. Conversely, when the initial hydrochloric acid/cysteine molar ratio increased to 1.0, it was confirmed that the ratio of the particle size of 40 to 80 mesh in the products rapidly decreased to about 40%, even when the final molar ratio was adjusted to 1.2.

### Example 4: Changes in Crystal Particle Size According to Final Hydrochloric Acid/Cysteine Molar ratio by Dual Adjustments of Molar ratio

In Example 3, L-cysteine hydrochloride hydrates were produced by varying the final hydrochloric acid/cysteine molar ratios, while fixing the initial hydrochloric acid/cysteine molar ratio at 0.85.

### (1) Concentration

The concentration was performed under a vacuum of 110 mmHg and an external temperature of 70°C until the concentration of L-cysteine reached 600 g/L. L-cysteine free form crystals were precipitated during the concentration.

### (2) Additional Hydrochloric Acid Addition

Hydrochloric acid was added to the L-cysteine crystal slurry solution so that the final molar ratio of hydrochloric acid/cysteine became 1.0 to 1.5.

### (3) Cooling and Recovery of Crystals

The thus-obtained hydrochloric acid mixture was cooled to 15°C at a constant cooling rate for 8 hours in a jacketed tank with stirring. Thereafter, L-cysteine crystals were separated into solid and liquid phases from the L-cysteine crystal slurry using a basket separator at a rotation speed of 3,000 rpm for 15 minutes. After separation, drying was performed using an oven dryer at 40°C for 2 hours or more to reduce the residual moisture to 0.5% or less, thereby finally producing L-cysteine hydrochloride hydrate crystals. Regardless of the hydrochloric acid/cysteine molar ratio, the purity of the L-cysteine hydrochloride monohydrate crystals was 99.0% or higher, and the particle size distribution is shown in Table 3 below.

**[Table 3]**

| Initial hydrochloric acid/cysteine molar ratio | Addition al hydrochl oric acid | Final hydrochloric acid/cysteine molar ratio | Product mesh | | |
|---|---|---|---|---|---|
| Eq. | Eq. | Eq. | -40 | 40 - 80 | 80 - |
| 0.85 | 0.15 | 1.0 | 29.3 | 66.7 | 4.0 |
| 0.85 | 0.25 | 1.1 | 40.2 | 56.6 | 3.2 |
| 0.85 | 0.35 | 1.2 | 43.9 | 52.1 | 4.0 |
| 0.85 | 0.65 | 1.5 | 56.5 | 40.0 | 3.5 |

Even when the hydrochloric acid/cysteine molar ratio was 1.2, the amount of crystals with a particle size of 40 to 80 mesh exceeded 50%. It was confirmed that adjusting the final hydrochloric acid/cysteine molar ratio during the concentration step or after the concentration step using the method described in the present disclosure, rather than obtaining L-cysteine hydrochloride hydrate crystals by adjusting the initial hydrochloric acid/cysteine molar ratio before concentration followed by concentration and cooling, allows for the production of L-cysteine hydrochloride hydrate crystals with an increased amount of crystals of 40 to 80 mesh particle size. This method maintains the recovery rate of L-cysteine hydrochloride crystals at 45% or more.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within metes and bounds of the claims, or equivalents of such metes and bounds are therefore intended to be embraced by the claims.

## Claims

1. A method for producing L-cysteine hydrochloride hydrate crystals having a controlled particle size, comprising:
(a) an initial hydrochloric acid addition step for adjusting the initial molar ratio of hydrochloric acid to L-cysteine (HCI/L-cysteine) by adding hydrochloric acid to a solution containing L-cysteine;
(b) a concentration step for concentrating the solution to which the hydrochloric acid has been added to obtain a concentrate;
(c) an additional hydrochloric acid addition step for adjusting the final molar ratio of hydrochloric acid to L-cysteine (HCI/L-cysteine) by additionally adding hydrochloric acid to the concentrate; and
(d) a recovering step for recovering L-cysteine hydrochloride hydrate crystals from the concentrate to which the hydrochloric acid has been additionally added.

2. The method of claim 1, wherein the L-cysteine hydrochloride hydrate crystals have a ratio of particles having a particle size of 40 to 80 mesh of 50% or more.

3. The method of claim 1, wherein the L-cysteine hydrochloride hydrate crystals have a ratio of particles having a particle size of 40 mesh or less of 60% or more.

4. The method of claim 1, wherein in the (a) initial hydrochloric acid addition step, hydrochloric acid is added to a solution containing L-cysteine such that the initial molar ratio of hydrochloric acid to L-cysteine (HCI/L-cysteine) is 0.5 or more and less than 1.0.

5. The method of claim 1, wherein in the (a) initial hydrochloric acid addition step, hydrochloric acid is added to a solution containing L-cysteine such that the initial molar ratio of hydrochloric acid to L-cysteine (HCI/L-cysteine) is 0.7 or more and less than 1.0

6. The method of claim 1, wherein in the (c) additional hydrochloric acid addition step, hydrochloric acid is additionally added to the concentrate such that the final molar ratio of hydrochloric acid to L-cysteine (HCI/L-cysteine) is 1.0 or more and less than 1.5.

7. The method of claim 1, wherein in the (c) additional hydrochloric acid addition step, hydrochloric acid is additionally added to the concentrate such that the final molar ratio of hydrochloric acid to L-cysteine (HCI/L-cysteine) is 1.0 or more and less than 1.2.

8. The method of claim 1, further comprising a purification step of obtaining a separated solution by injecting a fermentation solution containing L-cysteine into a continuous chromatography device prior to the initial hydrochloric acid addition step.

9. The method of claim 1, wherein the (b) concentration step is performed such that the concentration of L-cysteine in the solution is 500 to less than 700 g/L.

10. The method of claim 1, further comprising a cooling step for cooling the concentrate to which the hydrochloric acid has been additionally added between the (c) additional hydrochloric acid addition step and the (d) recovering step.

11. The method of claim 10, wherein in the cooling step, the concentrate is cooled to a temperature of 10 to 30°C at a rate of 3°C/hr or more and less than 10°C/hr.

12. L-cysteine hydrochloride hydrate crystals produced by the production method of any one of claims 1 to 11.
